**Europäisches Patentamt**

(19) **European Patent Office**

**Office européen des brevets**

(11) Numéro de publication: **0 043 596**
**B1**

(12) # FASCICULE DE BREVET EUROPÉEN

(45) Date de publication du fascicule du brevet:
05.11.86

(51) Int. Cl.⁴: **A 61 K 7/48**

(21) Numéro de dépôt: **81200468.7**

(22) Date de dépôt: **04.05.81**

(54) Procédé de contrôle de l'effet hydratant d'un produit à appliquer sur la peau.

(30) Priorité: **09.07.80 CH 5242/80**

(43) Date de publication de la demande:
**13.01.82 Bulletin 82/2**

(45) Mention de la délivrance du brevet:
**05.11.86 Bulletin 86/45**

(84) Etats contractants désignés:
**AT BE DE FR GB IT LU NL SE**

(56) Documents cité:
FR-A-2 269 327
LU-A-78 679

**Ralph G. Harry, Modern Cosmeticology"The Principles and Practice of Modern Cosmetics", Volume 1, 1962, pages 128-131
Marcus Karel, Owen R. Fennema, Daryl B. Lund, Principles of Food Science, Part II Physical Principles of Food Preservation, page 147
Thomas B. Fitzpatrick, Dermatology in General Medicine "Biology and Physiopathology of Skin", 1971
L. Acker, K.G. BERGNER, W. DIEMAIR, W. HEIMANN, F. KIERMEIER, J. SCHORMÜLLER, S.W. SOUCI, Handbuch der Lebensmittelchemie, Band V/2. Teil, 1968, Seite 39**

(73) Titulaire: **LABIOL S.A., 14, rue Grenus, CH- 1211-Geneve 1 (CH)**

(72) Inventeur: **Brun, Nicole, 14, rue Grenus, CH- 1211 Geneve 1 (CH)**

(74) Mandataire: **Meylan, Robert Maurice, c/o Bugnion SA Conseils en Propriété Industrielle 10, Route de Florissant Case Postale 375, CH- 1211 Genève 12 - Champel (CH)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

## Description

La présente invention concerne un procédé de contrôle de l'effet hydratant d'un produit à appliquer sur la peau humaine ou animale, tel que crème, pommade, gel, lait, onguent, poudre, lotion, solution, savon, détergent, schampooing, teinture et d'une manière générale toutes substances, composées ou simples, pouvant entrer en contact avec le tégument cutané.

On connaît différentes méthodes pour mettre en évidence et évaluer l'effet hydratant sur la peau de crèmes cosmétiques et pharmaceutiques ou de substances isolées. La plupart de ces méthodes utilisent la propriété de la kératine humaine ou animale, telle qu'on la rencontre par exemple dans la couche cornée de la peau, d'absorber de l'eau et de conserver son taux d'humidité. Ces mesures peuvent être faites in vitro ou aussi in vivo et mettent en jeu des techniques gravimétriques, photométriques, électriques, électroniques, etc. Ces méthodes et ces procédés sont appliqués en laboratoire et nécessitent un personnel spécialisé et des appareils sophistiqués. Elles ne peuvent pas être pour la plupart comprises facilement du consommateur et ne présentent pas d'aspect spectaculaire. Une méthode consistant à utiliser un épiderme de porc est décrite par exemple dans le brevet FR 2 269 327. L'épiderme doit être prélevé immédiatement après l'abattage, préparé et conservé à - 20° C jusqu'à utilisation. Le test nécessite des appareils de laboratoire.

La présente invention a pour but de permettre d'effectuer un contrôle de l'effet hydratant d'un produit d'une manière simple, économique, facilement compréhensible et de plus spectaculaire.

Procédé de contrôle de l'effet antidéshydratant sur la peau d'un produit cosmétique; par comparaison de deux échantillons dont l'un est traité avec le produit contrôle et l'autre est un témoin caractérisé en ce que le produit à tester est appliqué sur un fruit ou un légume et que le témoin est un fruit ou un légume identique à celui qui est traité: même cueillette, et de poids approximativement égal; ensuite on entrepose les échantillons dans des conditions identiques et on compare l'évolution de leur état de surface.

Le principe de l'invention est basé sur le fait que l'hydratation de la peau n'est pas un phénomène actif, c'est-à-dire un apport d'eau par le produit dit hydratant, tel qu'on l'a conçu au début, mais une diminution de la perte en eau par la peau dans le milieu ambiant.

Les fruits et légumes choisis pour un même contrôle seront de préférence non seulement de même espèce, mais aussi de même provenance, de la même cueillette et de poids approximativement égaux. Quelles que soient les conditions dans lesquelles ces végétaux sont entreposés pour un contrôle, ces conditions devront être égales pour tous ceux d'un même contrôle. Le choix des végétaux témoins et des végétaux à traiter se fait par tirage au sort. L'entreposage peut avoir lieu soit dans un local, soit à l'extérieur, soit encore dans une enceinte thermostatisée.

On peut utiliser plusieurs méthodes pour juger de l'effet hydratant ou plus précisément de l'effet antidéshydratant. La méthode la plus simple est l'observation jour après jour, de visu ou par photographie, des fruits et légumes traités et non traités. On voit, selon le végétal utilisé, que la surface du végétal non traité devient rapidement ridée, craquelée, plissée. En comparaison, si le végétal est traité avec un produit ayant un effet hydratant efficace, la surface reste lisse et sa peau tendue.

On peut confirmer cet effet et le mesurer d'une façon quantitative simplement par pesées successives à intervalles de temps déterminés, des fruits et légumes traités et non traités. On peut ainsi établir l'évolution de ces végétaux.

En général le traitement ne se fera qu'une seule fois, soit le premier jour, après quoi les végétaux entreposés ne seront touchés que pour la pesée. On peut toutefois le répéter plusieurs fois sur le même spécimen pour contrôler l'effet d'une application répétée correspondant par exemple à un traitement hebdomadaire ou journalier.

Le procédé de contrôle, selon l'invention, permet de tester l'effet hydratant d'un produit en fonction des différents climats ou situations géographiques, en particulier selon la température, l'altitude, la latitude, l'hygrométrie, l'ensoleillement.

Les fruits et légumes susceptibles d'être utilisés pour la mise en oeuvre de ce procédé sont nombreux et il est impossible d'en dresser ici une liste exhaustive. On utilisera avantageusement des fruits, en particulier des pommes et des poires pour les raisons suivantes: on les trouve toute l'année à un prix abordable; leur peau est relativement mince et perméable à la vapeur d'eau et dans des conditions ambiantes correspondant à celle d'un logement ou d'un bureau, la perte en eau de ces fruits non traités est objectivable rapidement par pesée, dans un délai de quelques jours à quelques semaines; la minceur de la peau de ces fruits fait qu'ils se rident rapidement lorsqu'ils se déshydratent et qu'il est ainsi facile de juger de visu de leur état de déshydratation.

Parmi les fruits, on pourra également utiliser les oranges, les mandarines et autres agrumes, les bananes, les pêches, les prunes, les abricots, les tomates et d'une façon générale tous les fruits qui changent d'aspect externe et perdent du poids lorsqu'ils se déshydratent.

Il est également possible d'utiliser des légumes tels que les pommes de terre, courgettes, concombres, raves, carottes et d'une manière générale tous les légumes qui changent d'aspect externe et perdent du poids lorsqu' ils se déshydratent.

Les fruits et légumes sont très sensibles à un effet irritant et lorsque le produit contrôlé par ce procédé n'est pas bien supporté par le tégument, le végétal réagit la plupart du temps en changeant de teinte ou en se flétrissant. Ainsi la méthode peut, en plus de l'effet hydratant, renseigner sur l'effet irritant ou toxique pour le tégument de la substance contrôlée. En résumé, le procédé selon l'invention présente les avantages suivants:

Le procédé est bon marché, il ne nécessite aucun appareillage, ni personnel spécialisé, il peut donc être utilisé directement par le consommateur.

Le résultat peut être constaté de visu, sans manipulation, ni mesure.

Le résultat peut être traduit quantitativement par pesée.

Le procédé permet simultanément de juger de l'effet irritant ou toxique des produits examinés sur le tégument.

Le procédé utilisant des végétaux comme organisme test fait que l'on évite ainsi des tests sur les animaux.

Enfin, le résultat est spectaculaire et se prête bien à la publicité et au marketing.

L'invention est illustrée au moyen de quelques exemples:

### Exemple 1:

examen d'une crème hydratante

Dix pommes type Golden delicious, en parfait état (sans marques de coups, peau intacte), sont rincées soigneusement à l'eau froide pour éliminer les poussiéres et impuretés, puis séchées délicatement à l'aide d'un linge. Les dix pommes sont réparties en deux groupes de cinq par tirage au sort. Chaque pomme est numérotée et pesée. Chaque pomme du groupe à traiter reçoit une application de crème H comme pour enduire une main, c'est-à-dire env. 1,5 g de crème, répartie sur toute la surface, sans oublier le réceptable de la queue. Les dix pommes sont placées côte à côte dans une pièce à l'abri des courants d'air et des rayons directs du soleil, dans des conditions de température et d'humidité correspondant à celles d'un appartement normal. On les photographie le premier jour, puis une fois par semaine. On les pèse tous les deux jours. Après 28 jours les pommes enduites de crème H n'ont pas changé d'aspect et leur poids n'a diminué en moyenne que de 8,5 %. Par contre, les pommes du groupe non traité sont ridées, ratatinées et leur poids a diminué en moyenne de 21 %.

### Exemple II:

examen d'un lait hydratant

Douze poires William sont réparties, aprés rinçage et séchage soigneux, en deux groupes de six. On enduit d'un lait hydratant G celles du groupe à traiter. On pèse les douze poires après une heure, puis on les dépose dans un local à l'abri des courants d'air et des rayons directs du soleil. On photographie les poires le premier jour, puis on les pèse et les photographie toutes les deux semaines. Après six semaines, les poires du groupe traité commencent à se rider très légèrement en surface, tandis que celles du groupe de comparaison sont déjà très ridées et ratatinées. Le poids moyen du groupe traité a diminué de 14,1 %, alors que le poids moyen du groupe de comparaison a baissé de 27,8 %.

### Exemple III:

comparaison de l'activité antidéshydratante de deux crémes

Douze pommes type Reinette du Canada, de belle qualité, à peau intacte et ne portant pas de marques de coups, sont rincées à l'eau froide et séchées. On les répartit en trois groupes de guatre par tirage au sort, après les avoir numérotées. Les pommes du groupe I sont enduites d'une crème M pour le visage, celles du groupe II d'une crème F vantée comme "super-hydratante" par sa publicité. Les pommes du groupe III ne sont pas traitées. Les douze pommes sont pesées 1h30 après le traitement, et photographiées. Elles sont ensuite entreposées côte à côte à l'extérieur, mais à l'abri des intempéries et des rayons solaires. Les températures varient au maximum entre 6° et 22°. Les pommes sont pesées tous les 5 jours et photographiées chaque semaine. Après 5 semaines, les pommes du groupe III sont fortement ratatinées, elles ont perdu en moyenne 22,7 % de leur poids. Celles du groupe II sont presqu'aussi ridées que celles du groupe III, elles ont perdu en moyenne 20,4 % de leurs poids initial. Par contre, celles du groupe I ne sont presque pas ridées et n'ont perdu que 12,6 % de leur poids en moyenne. Ce qui prouve une grande différence d'activité antidéshydratante entre les crèmes M et P, P possédant un effet proche de zéro alors que M a une activité certaine, puisqu'elle réduit les pertes en eau par les pommes de près de 50 %.

### Revendication

1. Procédé de contrôle de l'effet anti-déshychatant sur la peau d'un produit cosmetique; par comparaison de deux échantillons dont l'un est traité avec le produit contrôlé et l'autre est un témoin caractérisé en ce que le produit à tester est appliqué sur un fruit ou un légume et que le témoin est un fruit ou un légume identique à alun qui est traité: même cueillette, et de poids approximativement égal; ensuite on entrepose les échantillons dans des conditions identiques et on compare l'évolution de leur état de surface.

2. Procédé de contrôle selon la revendication 1, caractérisé en ce qu'on compare le poids des premier et deuxième spécimens après entreposage.

3. Procédé de contrôle selon la revendication 1, caractérisé en ce qu'on procède à des pesées des premier et deuxième spécimens à intervalles déterminés et compare l'évolution des poids mesurés.

4. Procédé de contrôle selon la revendication 1, caractérisé en ce qu'on répète l'opération plusieurs fois sur le même spécimen pour contrôler l'effet d'une application répétée.

**Patentansprüche**

1. Verfahren zur Prüfung der eine Austrocknung verzögerden Wirkung eines kosmetischen Produktes auf die Haut durch Vergleich zweier Proben, von denen die eine mit dem zu prüfenden Produkt behandelt ist und die andere zum Vergleich dient, dadurch gekennzeichnet, dass das zu prüfende Produkt auf mindestens eine Frucht oder ein Gemüse aufgebracht wird und dass die Vergleichsprobe mindestens eine gleiche Frucht oder ein gleiches Gemüse ist wie die beziehungsweise das behandelte, und zwar auch hinsichtlich der Ernte und etwa gleichem Gewicht, worauf man diese Proben unter gleichen Bedingungen lagert und die Veränderung ihres Oberflächenzustandes vergleicht.

2. Verfahren nach Anspruch 1, dadurch gekennzeichnet, dass man das Gewicht der ersten und zweiten Proben nach der Lagerung vergleicht.

3. Verfahren nach Anspruch 2, dadurch gekennzeichnet, dass man das Auswiegen der ersten und zweiten Proben in bestimmten Zeitabständen ausführt und die Veränderung der gemessenen Gewichte vergleicht.

4. Verfahren nach Anspruch 1, dadurch gekennzeichnet, dass man den Vorgang mehrere Male an derselben Probe wiederholt, um die Wirkung einer wiederholten Anwendung zu prüfen.

**Claims**

1. Method of ascertaining the anti-dehydrating effect of a cosmetic product on the skin by comparing two samples, one sample being treated with the product to be tested and the other sample being used for comparison, characterized in that the product to be tested is applied on a fruit or a vegetable and that the sample used for comparison is a fruit or vegetable identical to the sample to be tested, from the same gathering and having approximately the same weight, that both samples are stored under the same storage conditions and that the evolution of the surface condition of the samples is compared.

2. The method of claim 1, characterized in that the weights of the first and second samples are compared at the end of the storage period.

3. The method of claim 1, characterized in that the first and second samples are weighed at predetermined time intervals and the evolution of the measured weights is compared.

4. The method of claim 1, characterized in that the steps are repeated several times on the same sample to determine the effect resulting from a repeated application of the product to be tested.